(19) Europäisches Patentamt
European Patent Office
Office européen des brevets

(11) Publication number: **0 100 569**
**B1**

(12) **EUROPEAN PATENT SPECIFICATION**

(45) Date of publication of patent specification: **04.06.86**

(51) Int. Cl.$^4$: **C 07 C 97/065,** A 61 K 31/135

(21) Application number: **83200987.2**

(22) Date of filing: **04.07.83**

# (54) Spasmolytically active (+)S-enantiomer of secoverine.

(30) Priority: **12.07.82 NL 8202810**

(43) Date of publication of application:
**15.02.84 Bulletin 84/07**

(45) Publication of the grant of the patent:
**04.06.86 Bulletin 86/23**

(84) Designated Contracting States:
**AT BE CH DE FR GB IT LI LU NL SE**

(56) References cited:
**US-A-4 125 623**

**ARZNEIMITTELFORSCHUNG, vol. 30, no. 9, 1980 AULENDORF (DE) J.M.A. ZWAGEMAKERS et al.: "Pharmacology of Secoverine, a New Spasmolycric Agent with Specific Antimuscarinic Properties", pages 1517-1526**

**E. J. ARIENS (Editor), Drug desin Vol. 1, Academic Press, 1971**

(73) Proprietor: **DUPHAR INTERNATIONAL RESEARCH B.V**
**C.J. van Houtenlaan 36**
**NL-1381 CP Weesp (NL)**

(72) Inventor: **Hartog, Jan**
**c/o OCTROOIBUREAU ZOAN B.V.**
**Apollolaan 151**
**NL-1077 AR Amsterdam (NL)**
Inventor: **Zwagemakers, Johannes Maria Antonius**
**c/o OCTROOIBUREAU ZOAN B.V.**
**Apollolaan 151**
**NL-1077 AR Amsterdam (NL)**
Inventor: **van Wijngaarden, Ineke**
**c/o OCTROOIBUREAU ZOAN B.V.**
**Apollolaan 151**
**NL-1077 AR Amsterdam (NL)**

(74) Representative: **Muis, Maarten et al**
**OCTROOIBUREAU ZOAN B.V. P.O. Box 140**
**NL-1380 AC Weesp (NL)**

**EP 0 100 569 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a method of preparing pharmaceutical compositions which comprise a spasmolytically active derivative of 1-cyclohexyl-4-[ethyl(p-methoxy-α-methylphenethyl)amino]-1-butanone (known as secoverine), or a salt thereof, as an active substance, and to the compositions thus obtained. The invention furthermore relates to a method of preparing the (+) -enantiomer of 1-cyclo-hexyl-4-[ethyl-(p-methoxy-α-methylphenethyl)amino]-1-butanone or a salt thereof, and to the (+)-enantiomer thus to be obtained and the salts thereof.

It is known from Netherlands Patent Application 7404733 that a group of compound of which secoverine forms part has a specific, spasmolytic activity on the smooth musculature of the tractus gastro-intestinalis, the tractus urogenitalis and the bronchial system.

Further it is known from Arzneimittelforschung, Vol. 30, no. 9, (1980), pages 1517—1526 that racemic 1-cyclo-hexyl-4-[ethyl(p-methoxy-α-methylphenethyl)amino]-1-butanone hydrochloride (i.e. secoverine hydrochloride) is a neurotropic agent with specific antimuscarinic properties.

It has now been found that the anticholinergic spasmolytic activity in peripheral organs of the (+)-enantiomer of secoverine is approximately twice as strong as that of the racemate.

Although it is known (Burger, Medicinal Chemistry, pages 98—100) of some anticholinergics having an optically active carbon atom in the molecule, that the entantiomers have a different activity, this was not known of secoverine.

It is surprising that the found difference in activity in peripheral organs between the (+)- and (−)-enantiomers of secoverine is so large: the (+)-enantiomer of secoverine is approximately 100 times as active as the (−)-enantiomer.

It has furthermore been found that, in contrast with what might be expected on the basis of the 100 times larger activity of (+)-enantiomer of secoverine with respect to the (−)-enantiomer, there is no or substantially no difference between the two enantiomers as regards the noradrenaline (NA)-re-uptake-inhibiting and NA-potentiating properties. As regards the less desirable local anaesthetic properties, the (−)-enantiomer is clearly more active in view of the (+)-enantiomer.

The advantages of the twice as strong peripheral activity and the approximately equal to smaller undesired side effects of the (+)-enantiomer of secoverine in comparison with the racemate are:

1) only half the dose is necessary to obtain the same therapeutic effect;

2) that as a result of this the non-desired side effects are halved at least.

In a few tests suitable for this purpose, the spasmolytic activity of the (+)-enantiomer of secoverine was compared both in vitro and in vivo with that of the racemate and/or the (−)-enantiomer.

a) Peripheral spasmolytic activity in vitro

These tests were carried out according to the normal 4-point analysis in which the terminal guinea pig ileum was used as isolated organ and carbachol was used as a cholinergic spasmogen.

The required organs were isolated from killed guinea pigs and the contents and adherent tissues were carefully removed from suitable pieces. The said organs were then suspended in a bath containing 50 ml of Tyrode solution through which oxygen was led. The spasmogenically active substance (carbachol) was added to the bath intermittently and, after a constant response to the spasmogen had been obtained, the spasmolytic activity of the substances to be tested was measured by adding them to the bath 30 seconds prior to the spasmogen.

The relative activity of the (+)-enantiomer of secoverine as compared with the racemate was calculated from a series of 4-point assays: the (+)-enantiomer of secoverine was 1.86 times as active as the racemate.

Similar results were obtained by using rat jejunum as an organ and using furtrethonium as a spasmogenically active substance: the (+)-enantiomer of secoverine now was 2.10 times as active as the racemate.

b) Affinity to the muscarine receptor

According to the method of J.M. van Rossum, Arch. Int. Pharmacodyn, *143* (1963) 299 cumulative dose-response curves were determined. For this purpose, rat jejunum was used as an organ and furtrethonium was used as a spasmogenically active substance. Affinity constants were expressed as $pA_2$-values.

Both enantiomers of secoverine and the racemate gave a good shift to the right of the dose response curves of furtrethonium without the maximum of the curves being changed:

| compound | $pA_2$ |
|---|---|
| (+)-enantiomer of secoverine | 8.10 |
| racemate | 7.90 |
| (−)-enantiomer of secoverine | 5.88 |

It may be concluded from these results that the (+)-enantiomer of secoverine has a more than 100 times larger activity than the (−)-enantiomer.

c) Anticholinergic activity in vivo

These tests were carried out according to the method by J.M.A. Zwagemakers, Arzneimittelforschung, *30* (II), (1980), 1517—1526 in which carbachol was used as a spasmogenically active substance. After a constant response to the spasmogen had been obtained, the compound to be tested was administered intravenously (i.v.) to guinea pigs 3 minutes prior to the next quantity of spasmogenically active substance. The activity of the compounds was determined for 52 minutes. Contractions obtained after preceding administration of the substance to be tested were expressed as percentages of the average of two control values and were plotted graphically. The average percentage of inhibition during the period of 52 minutes was calculated from the area under the curve, and the maximum inhibition and the duration of the spasmolytic activity were estimated by means of the time-contraction curve. The spasmolytic activity is considered to be terminated when two successive contractions show less than 20% inhibition. The following results were obtained:

| compound | Average % of total inhibition | Average % of max. inhibition | Duration of act. in min. |
|---|---|---|---|
| (+)-enantiomer of secoverine 0.05 mg/kg/i.v. | 51 | 69 | >52 |
| (+)-enantiomer of secoverine 0.10 mg/kg/i.v. | 88 | 93 | >52 |
| Racemate 0.10 mg/kg/i.v. | 55 | 69 | >52 |

It appears from the results that the (+)-enantiomer of secoverine is about twice as active as the racemate.

d) Potentiation of the activity of noradrenaline on the vas deferens of the rat in vitro

The test used is a modification of the method as described by Ursillo and Jacobsen, J. Pharmacol. Ex. Ther. *148*, (1965), 247.

A significant noradrenaline potentiation was found both of the racemate and of the (+)- and (+)-enantiomer. The smallest dose which gave a potentiating effect was $1 \times 10^{-7}$M, and the potentiating activity was maximum in a quantity of $5 \times 10^{-7}$M. Higher dosages caused a non-specific decrease of the maximum of the noradrenaline curve. In addition to a significant shift to the left, the noradrenaline curves obtained under the influence of secoverine or one of the enantiomers, showed a rather strong increase of the maximum contraction.

For quantifying the effect at the 50% point of the contraction curve (maximum of the control noradrenaline curve = 100%), the shift of the curve obtained with the compound to be tested (expressed as DR) was measured. The $pP_2$-values were calculated in the same manner as the $pA_2$-values mentioned sub b), according to the equation

$$pP_2 = -\log[\text{secoverine}] + \log(DR—1).$$

Hence the $pP_2$-value is the negative logarithm of the molar concentration which gives a shift to the left of a factor 2.

| compound | $pP_2$-value |
|---|---|
| racemate | 6.51 |
| (+)-enantiomer of secoverine | 6.58 |
| (−)-enantiomer of secoverine | 6.77 |

e) Effect on the synaptosomal re-uptake of noradrenaline

In this test a suspension of rat hypothalamus synaptosomes was used. The re-uptake inhibition was expressed in $pI_{50}$-values, i.e. the negative logarithm of the molar concentration which gives 50% inhibition:

| compound | $pI_{50}$ |
|---|---|
| (+)-enantiomer of secoverine | 4.6 |
| racemate | 5.0 |
| (−)-enantiomer of secoverine | 5.0 |

f) local anaesthetic activity

The anaesthetic activity on the corona of male mice (18—22 g), 10 animals per dose, was determined according to the method by Weidmann and Petersen, J. Pharmacol. Exp. Ther., *115* (1955), 246. The concentration of the solution of the compounds which gave local anaesthesia in 50% of the animals was determined graphically ($EC_{50}$-value):

| compound | $EC_{50}$-value (% w/vol) |
|---|---|
| (+)-enantiomer of secoverine | 0.20 |
| racemate | 0.13 |
| (−)-enantiomer of secoverine | 0.08 |

It appears from test d) that there is no difference between the racemate and the two enantiomers as regards the noradrenaline potentiating activity. It appears from tests e) and f) that the (+)-enantiomer of secoverine has a noradrenaline re-uptake inhibiting and local anaesthetic activity which is substantially weaker than those of the (−)-enantiomer. This means that the much stronger spasmolytic activity of the (+)-enantiomer of secoverine is not related with a proportional increase of undesired side effects. The (+)-enantiomer of secoverine hence is a much more specific spasmolytic than the racemate.

The (+)-enantiomer of secoverine can be prepared by methods known per se, by synthesis from optically active intermediates and by resolution of the racemate into its antipodes.

A suitable method for example is the reaction of (+)-2-(4-methoxyphenyl)-1-methyldiethylamine with a compound of the formula

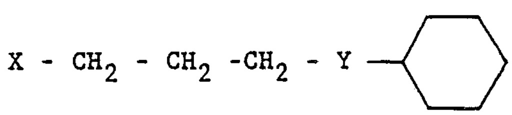 ,

wherein X represents a leaving group, and Y represents a protected keto group. Suitable leaving groups are for example halogen, or the group —OR, wherein R is e.g. a tosyl- or a triflate group. Examples of suitable protecting groups are alkylene ketals, particularly ethylene ketal.

The reaction is preferably carried out in an inert solvent such as for example acetonitrile, dimethylformamide, benzene, toluene, acetone and methyl ethyl ketone. The reaction mixture may contain an acid binding agent such as a tertiary amine like triethylamine and pyridine, or an inorganic base such as potassium carbonate. The reaction temperature is between 0 and 200°C.

The protecting group can be removed by means of a hydrolysis with diluted acids such as hydrochloric acid or sulfuric acid, optionally after mixing with ether of acetone, at temperatures between room temperature and the boiling point of the solvent used.

Another suitable method for preparing the (+)-enantiomer of secoverine is based on the resolution of the racemate into its optically active antipodes by means of known techniques, for example through fractionated crystallization of diastereomeric salts of the racemic base with an optically active acid, such as for example (+)-camphorsulfonic acid, (−)-mandelic acid, (+)- or (−)-binaphtylphosphoric acid, and so on.

The preparation of the (+)-enantiomer of secoverine will now be described in greater detail with reference to the ensuing specific example.

Example

(+)S-1-cyclohexyl-4-[ethyl(p-methoxy-α-methylphenethyl)-amino]-1-butanone hydrochloride.

A solution of 1.4 g (7.2 mmol) of (+)-2-(4-methoxyphenyl)-1-methyl diethylamine (see Rec. trav. chim. *85,* (1966), 612) 2.1 g (9.0 mmol) of ethylenecycloacetal of 4-chloro-1-cyclohexyl-1-butanone, 0.7 g (6.9 mmol) of triethylamine and 1.3 g (7.8 mmol) of potassium iodide in 8 ml of dimethyl formamide was heated while stirring at a temperature of 75—80°C for 24 hours.

After cooling to room temperature, 150 ml of ether were added and the reaction mixture was washed successively with a 5% soda solution and water.

The formed amino-acetal was hydrolyzed by adding 1 to 2 ml of concentrated hydrochloric acid to the wet ether layer until pH 2—3, after which the inhomogeneous mixture was stirred at room temperature for 3 hours.

The mixture was then evaporated under reduced pressure and the residue was taken up in 50 ml of water. The resulting solution was washed a few times with 25 ml ether portions, then made basic with crystal soda until pH 9, and then extracted with ether.

The resulting crude base of the (+)-enantiomer of secoverine (1.7 g) was purified chromatographically over a silica gel column, in which chloroform and chloroform-methanol (50:1) were successively used as an eluent.

The collected desired fractions were evaporated under reduced pressure, taken up in 10 ml of absolute ethanol and acidified with 2 N alcoholic hydrochloric acid. After evaporating again, evaporating twice with toluene, and crystallization from an acetone-ether mixture, 1.2 g (3.1. mmol) of the (+)-enantiomer of secoverine having a melting-point of 101—102°C, and $[\alpha]_D^{20} = +19.1$ ($H_2O$) were obtained.

**Claims**

1. A method of preparing pharmaceutical compositions by bringing a spasmolytically active derivative of 1-cyclohexyl-4-[ethyl(p-methoxy-α-methylphenethyl)amino]-1-butanone or a salt thereof, into a form suitable for administration, characterized in that (+)-S-1-cyclohexyl-4-[ethyl-(p-methoxy-α-methylphenethyl)amino]-1-butanone or a salt thereof is used as an active substance.

2. Pharmaceutical compositions obtained according to the method as claimed in Claim 1.

3. A method of preparing an enantiomer of 1-cyclohexyl-4-[ethyl(p-methoxy-α-methylphenethyl)amino)-1-butanone or a salt thereof, characterized in that (+)-S-1-cyclohexyl-4-[ethyl(p-methoxy-α-methylphenethyl)amino]-1-butanone or a salt thereof is prepared by converting (+)-1-(4-methoxyphenyl)-1-methyldiethylamine with a compound of the formula

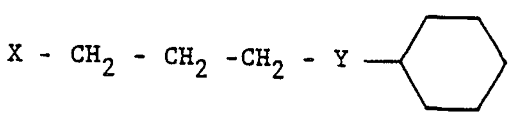

,

wherein X represents a leaving group, and Y is a protected keto group.

4. Process according to claim 3, characterized in that the racemate of 1-cyclohexyl-4-[ethyl(p-methoxy-α-methylphenethyl)amino]-1-butanone is resolved by a method known for analogous compounds.

5. (+)-S-1-cyclohexyl-4-[ethyl-(p-methoxy-α-methylphenethyl)amino]-1-butanone and salts thereof.

**Patentansprüche**

1. Verfahren zum Herstellen von pharmazeutischen Zusammensetzungen, indem ein spasmolytisch wirksames Derivat von 1-Cyclohexyl-4-(äthyl-(p-methoxy-α-methylphenäthyl)-amino]-1-butanon oder ein Salz davon in eine zur Verabreichung geeignete Form gebracht wird dadurch gekennzeichnet, daß (+)-S-1-Cyclohexyl-4-[äthyl-(p-methoxy-α-methylphenäthyl)-amino]-1-butanon oder ein Salz davon als Wirkstoff verwendet wird.

2. Pharmazeutische Zusammensetzungen, erhalten nach dem Verfahren, wie in Anspruch 1 beansprucht.

3. Verfahren zum Herstellen eines Enantiomers von 1-Cyclohexyl-4-[äthyl-(p-methoxy-α-methylphenäthyl)-amino]-1-butanon oder eines Salzes davon, dadurch gekennzeichnet, daß (+)-S-1-Cyclohexyl-4-[äthyl-(p-methoxy-α-methylphenäthyl)-amino]-1-butanon oder ein Salz davon durch Überführen von (+)-1-(4-Methoxyphenyl)-1-methyldiäthylamin mit einer Verbindung der Formel

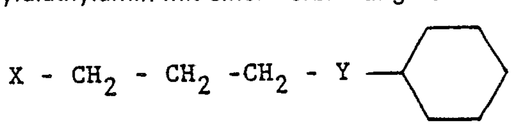

,

worin X eine abspaltbare Gruppe darstellt und Y eine geschützte Ketogruppe ist, hergestellt wird.

4. Verfahren nach Anspruch 3, dadurch gekennzeichnet, daß das Racemat von 1-Cyclohexyl-4-[äthyl-(p-methoxy-α-methylphenäthyl)-amino]-1-butanon durch ein für analoge Verbindungen bekanntes Verfahren getrennt wird.

5. (+)-S-1-Cyclohexyl-4-[äthyl-(p-methoxy-α-methylphenäthyl)-amino]-1-butanon und Salze davon.

**Revendications**

1. Procédé pour préparer des compositions pharmaceutiques, en transformant un dérivé spasmolytiquement actif de la 1-cyclohexyl-4-[éthyl(p-méthoxy-α-méthylphénétyl)amino]-1-butanone ou d'un de ses sels en une forme convenant à l'administration, caractérisé en ce qu'on utilise en tant que substance active la 1-cyclohexyl-4-[éthyl-(p-méthoxy-α-méthylphénéthyl)amino]-1-butanone-(+)-S ou l'un de ses sels.

2. Compositions pharmaceutiques obtenues par le procédé selon la revendication 1.

3. Procédé pour la préparation d'un énantiomère de la 1-cyclohexyl-4-[éthyl(p-méthoxy-α-méthylphénéthyl)-amino]-1-butanone ou d'un de ses sels, caractérisé en ce qu'on prépare la 1-cyclohexyl-4-[éthyl(p-méthoxy-α-méthylphénéthyl)amino]-1-butanone(+)-S ou l'un de ses sels en convertissant la 1-(4-méthoxyphényl)-1-méthyldiéthylamine-(+) à l'aide d'un composé de formule

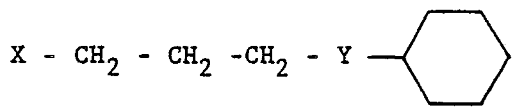 ,

dans laquelle X représente un groupe quittant la molécule, et Y est un groupe céto protégé.

4. Procédé selon la revendication 3, caractérisé en ce que le racémate de 1-cyclohexyl-4-[éthyl(p-méthoxy-α-méthylphénéthyl)amino]-1-butanone est résolu par un procédé connu pour des composés analogues.

5. 1-cyclohexyl-4-[éthyl-(p-méthoxy-α-méthylphénéthyl)-amino]-1-butanone-(+)-S et ses sels.